# EUROPEAN PATENT APPLICATION

(11) **EP 2 133 428 A1**
(43) Date of publication of application: **16.12.2009**
(21) Application number: 09011354.9
(22) Date of filing: 25.11.2004
(51) Int. Cl.: C12N 15/866, C12N 15/10, A61K 31/7088

(54) **New baculovirus vectors for improved protein production**

(30) Priority: 09.03.2004 US 552072 P
(62) Divisional of application: 04803271.8
(71) Applicant: Eidgenössische Technische Hochschule Zürich, 8092 Zürich (CH)
(72) Inventor: Berger, Imre, 8046 Zürich (CH); Fitzgerald, Daniel, J., 8049 Zürich (CH); Richmond, Timothy, J., 8049 Zürich (CH)
(74) Representative: Kasche, André

(57) **Abstract**

The present invention relates to a baculovirus vector, wherein the two baculoviral genes v-cath and chiA are functionally disrupted, as well as corresponding host cells, methods for producing recombinant proteins using said vectors and/or host cells, corresponding uses and a kit of parts comprising said baculoviruses and/or host cells.

## Description

The present invention relates to a baculovirus vector, wherein the two baculoviral genes v-cath and chiA are functionally disrupted, as well as corresponding host cells, methods for producing recombinant proteins using said vectors and/or host cells, corresponding uses and a kit of parts comprising said baculoviruses and/or host cells.

### Background of the invention

Recombinant baculoviruses are particularly attractive for high-level production of large protein assemblies (O'Reilly et al., Baculovirus expression vectors. A laboratory manual. Oxford Press, New York (1994)). Even architecturally complex particles such as capsid structures have been successfully assembled in insect cells using the baculovirus system (Roy et al. Baculovirus multigene expression vectors and their use for understanding the assembly process of architecturally complex virus particles. Gene 190, 119-129 (1997)). Expression of several genes in one cell can be achieved by co-infection with viruses carrying one foreign gene each. However, this approach inevitably leads to considerable variations in individual protein production levels as a result of statistical variance in virus stochiometry between infected cells. A superior alternative is infection with one baculovirus containing all heterologous genes of choice (Roy et al., see above; Bertolotti-Ciarlet et al., Immunogenicity and protective efficacy of rotavirus 2/6-virus-like particles produced by a dual baculovirus expression vector and administered intramuscularly, intranasally, or orally to mice. Vaccine 21, 3885-3900 (2003)). This is possible due to the flexible nature of the AcNVP envelope which allows for accommodation of large DNA insertions into the circular 130 kb dsDNA baculovirus genome.

It is the objective of the present invention to provide new and improved baculoviral tools and methods for recombinant protein production.

### Summary of the Invention

In a first aspect, the above-mentioned objective is solved by providing a baculovirus in general, wherein the two baculoviral genes v-cath and chiA are functionally disrupted.

When engineering baculovirus expression vectors, it was surprisingly found that the functional disruption of two baculoviral genes, v-cath and chiA, leads to improved maintenance of cellular compartiments during infection and protein expression. The quality of the proteins produced by such a novel baculovirus system is significantly improved through reduction of viral-dependent proteolytic activity and cell lysis. Furthermore, the disruption provides for the option of utilizing chitin-affinity chromatography for purification without interference with the *chiA* gene product. The *v-cath* gene encodes for a viral protease, V-CATH, which is activated upon cell death by a process dependent on a juxtaposed gene on the viral DNA, *chiA*, which encodes for a chitinase.

Preferably, vectors according to the present invention additionally comprise a site for SSRs (site specific recombinases), preferably LoxP for cre-lox site specific recombination.

More preferably, the cre-lox site is located in one or both of the two baculoviral genes v-cath and chiA and disrupts their function.

As used herein, the term "MultiBac" stands synonymously for the exemplary and preferred embodiment of the said baculovirus viral vector, i.e. a modified baculovirus genome lacking the genes for *v-cath* and *chiA*.

In another aspect, the present invention relates to a host cell comprising a vector according to the invention.

Preferred host cells for practicing the invention are those selected from the group consisting of mammalian cells, preferably human cells, rodent cells, porcine cells, bovine cells, ovine cells; *C*. *elegans* cells; yeast cells; insect cells; and *E. coli* cells.

Preferred yeast cells for practicing the invention are *S*. *cervisiae, S.pombe, C. albicans* and *P. pastoris*

Preferred *E*. *coli* cells for practicing the invention are Top10, DH5α, DH10α, HB101, TG1, BW23473, BW23474 cells.

Preferred insect cells for practicing the invention are *S*. *frugiperda* cells, preferably Sf9, Sf21, Express Sf+ or High Five H5 cells, and *D. melanogaster*, preferably S2 Schneider cells.

Preferred human cells for practicing the invention are selected from the group consisting of HeLa, Huh7, HEK293, HepG2, KATO-III, IMR32, MT-2, pancreatic β-cells, keratinocytes, bone-marrow fibroblasts, CHP212, primary neural cells, W12, SK-N-MC, Saos-2, WI38, primary hepatocytes, FLC4, 143TK-, DLD-1, embryonic lung fibroblasts, primary foreskin fibroblasts, Saos-2 osteosarcoma, MRC5, and MG63 cells.

In a further aspect, the present invention is directed to the use of a baculovirus vector and/or a host cell according to the invention for producing recombinant proteins.

The use of baculoviral vectors with functionally disrupted v-cath and chiA genes allows for improved maintenance of cellular compartments during infection and protein production. The quality of proteins produced by such a vector system is significantly improved through a reduction of viral dependent proteolytic activity and reduced cell lysis.

Of course, cells comprising a vector according to the present invention are by no means limited to isolated cells or cell tissues.

In another aspect, the present invention relates to a kit of parts comprising at least a vector and/or a host cell according to the invention, optionally also comprising an instruction manual, antibiotics, buffers, and/or marker compounds.

An additional aspect of the invention is directed to a method for producing recombinant proteins and/or vaccines comprising the step of culturing a baculovirus vector and/or host cell according to the invention.

### Description of the figures

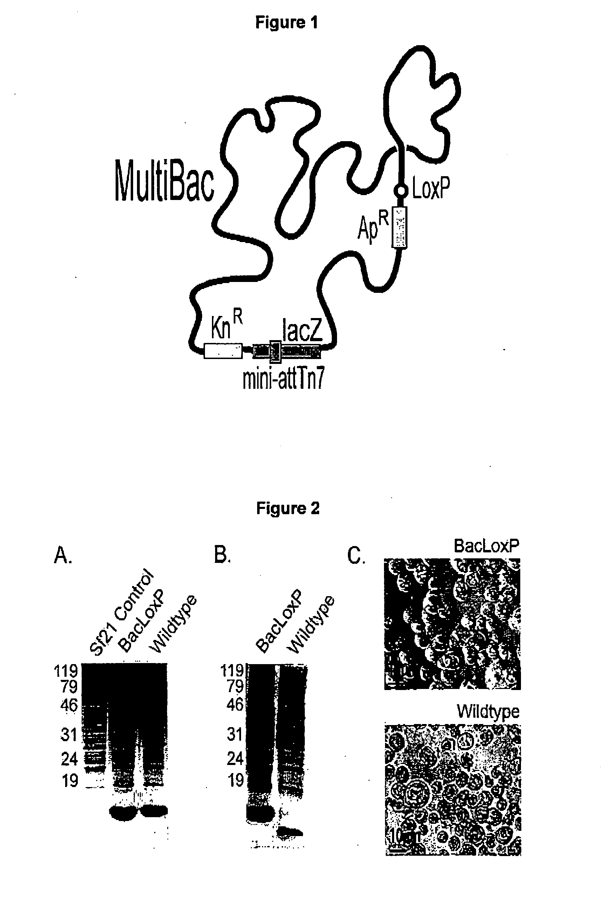

**Figure 1****: MultiBac baculoviral DNA.**
   The modified viral genome is shown in a schematic representation. The Tn7 attachment site is located within a LacZα gene; insertion of Tn7 elements from pFBDM derivatives therefore produces a white phenotype when plated on agar containing BluoGal and IPTG. In place of the disrupted *v-cath* and *chiA* viral genes, a LoxP sequence was inserted to accept pUCDM derivatives by Cre catalysis, producing a chloramphenicol resistant phenotype.
**Figure 2****: Effect of *v-cath* and *chiA* deletion in MultiBac.**
   Comparison of the lysate from cells infected with MultiBac virus according to Fig. 1 and a commercially available baculovirus ("Wildtype"). Viral-dependent protein production in both samples is virtually identical at 48 hours post infection (Fig. A). Incubation of cell lysate for 96 hours at RT after harvest shows a virtually unaltered protein content in the sample from cells infected with MultiBac. In contrast, proteolysis is evident in the sample from cells infected with wildtype virus (Fig. B). Effect of protease deletion on cell morphology at 72 hours post infection is shown in micrographs of cells infected with MultiBac lacking *v-cath* and *chiA* (MultiBac) or with commercial virus containing the protease and chitinase genes (Wildtype). Cells in the upper micrograph are uniformly round and appear intact. In contrast, cell lysis is prevalent in the lower micrograph (Fig. C). In the following specific embodiments of the invention will be described by way of example and these examples are not to be construed to be limiting to the scope of the present invention.

### Examples

In the following new baculovirus transfer vectors according to the present invention comprising an engineered baculovirus genome with improved protein production properties are described. Two baculoviral genes were disrupted which leads to improved maintenance of cellular compartments during infection and protein production. The quality of proteins produced by this system is significantly improved through a reduction of viral dependent proteolytic activity and reduced cell lysis.

### Example 1: Baculovirus engineered for improved protein production

The baculovirus genome was modified to obtain improved protein production properties. Two baculoviral genes, *v-cath* and *chiA*, were disrupted which leads to improved maintenance of cellular compartments during infection and protein production. The *v-cath* gene encodes for a viral protease, V-CATH, which is activated upon cell death by a process dependent on a juxtaposed gene on the viral DNA, *chiA*, which encodes for a chitinase. Both genes were disrupted to eliminate V-GATH activity and to gain the option of utilizing chitin-affinity chromatography for purification without interference from the *chiA* gene product. The quality of proteins produced by this so-called MultiBac baculovirus is significantly improved through a reduction of viral-dependent proteolytic activity and reduced cell lysis. In place of the disrupted viral DNA sequence, a LoxP sequence for *cre-lox* site-specific recombination was placed. For further details see Fig. 1.

### Example 2: Effect of chiA and v-cath deletion in MultiBac

During heterologous protein production using a commercially available baculovirus expression system, viral-dependent proteolytic breakdown consistent with the action of a cysteine protease was observed. The baculoviral *v-cath* gene encodes for a cysteine protease which is directly involved in liquefaction of the insect cell host. V-CATH is activated upon cell death by a process dependent on a juxtaposed gene on the viral DNA, *chiA*, which encodes for a chitinase. Disruption of both genes eliminated V-CATH activity and further provided the option of utilizing chitin-affinity chromatography for purification without interference from the *chiA* gene product. The effect of the disruption was assayed by comparing samples from cells infected with MultiBac virus to cells infected with a commercially available baculovirus carrying *v-cath* and *chiA*. A strongly reduced proteolytic activity, concomitant with much improved maintenance of cell integrity, was observed in lysate from cells infected with MultiBac (for more detail, see Fig. 2).

## Claims

1. A baculovirus vector, wherein the two baculoviral genes v-cath and chiA are functionally disrupted.

2. The baculovirus vector of claim 1 additionally comprising a site for SSRs (site specific recombinases), preferably LoxP for cre-lox site specific recombination.

3. The baculovirus vector of claim 2, wherein the cre-lox site is located in one or both of the two baculoviral genes v-cath and chiA and disrupts their function.

4. A host cell comprising a vector according to any of claims 1 to 3.

5. A method for producing recombinant proteins and/or vaccines comprising the step of culturing a baculovirus vector and/or a host cell according to any of claims 1 to 4.

6. Use of a baculovirus vector and/or a host cell according to any of claims 1 to 4 for producing recombinant proteins.

7. Kit of parts comprising a baculovirus and/or host cell according to any of claims 1 to 4.
